# EUROPEAN PATENT APPLICATION

(11) **EP 1 621 638 A1**
(43) Date of publication of application: **01.02.2006**
(21) Application number: 04731764.9
(22) Date of filing: 07.05.2004
(51) Int. Cl.: C12Q 1/68

(54) **METHOD OF ANALYZING GENE INTRODUCTION SITE**

(30) Priority: 07.05.2003 JP 2003129558; 07.07.2003 JP 2003192719
(71) Applicant: TAKARA BIO INC., Otsu-shi, Shiga 520-2193 (JP)
(72) Inventor: TOMONO, Jun, 7100845 (JP); UENO, Harumi;, Kusatsu-shi, Shiga 5250025 (JP); TAKEDA, Osamu, 5211124 (JP); MUKAI, Hiroyuki;, Moriyama-shi, Shiga 5240102 (JP); SAGAWA, Hiroaki, 5250025 (JP); KATO, Ikunoshin, 5291851 (JP)
(74) Representative: Schnappauf, Georg
(86) International application number: PCT/JP2004/006513
(87) International publication number: WO 2004/099446

(57) **Abstract**

A method of analyzing, in chromosomal DNA having a retroviral vector incorporated therein, DNA region derived from chromosome which is adjacent to the vector, **characterized in that** a primer extension reaction and a nucleic acid amplification reaction are carried out in the presence of a compound capable of lowering the Tm value of double stranded nucleic acid. There are further provided a kit and buffer for use in this method.

SEQUENCE LISTING

<110> TAKARA BIO INC.

<120> Method of analyzing gene introduction site

<130> 38-89

<140> EP 04 731 764.9
<141> 2004-05-07

<150> JP 2003-129558
<151> 2003-05-07

<150> JP 2003-192719
<151> 2003-07-07

<160> 10

<170> PatentIn Ver. 2.1

<210> 1
<211> 720
<212> DNA
<213> Artificial Sequence

<220>
<223> Description of Artificial Sequence: Gene encoding red-shifted
green fluorescence protein.

<400> 1

atggctagca aaggagaaga actcttcact ggagttgtcc caattcttgt tgaattagat  60

ggtgatgtta acggccacaa gttctctgtc agtggagagg gtgaaggtga tgcaacatac 120

ggaaaactta ccctgaagtt catctgcact actggcaaac tgcctgttcc atggccaaca 180

ctagtcacta ctctgtgcta tggtgttcaa tgcttttcaa gatacccgga tcatatgaaa 240

cggcatgact ttttcaagag tgccatgccc gaaggttatg tacaggaaag gaccatcttc 300

ttcaaagatg acggcaacta caagacacgt gctgaagtca agtttgaagg tgataccctt 360

gttaatagaa tcgagttaaa aggtattgac ttcaaggaag atggaaacat tctgggacac 420

aaattggaat acaactataa ctcacacaat gtatacatca tggcagacaa acaaaagaat 480

ggaatcaaag tgaacttcaa gacccgccac aacattgaag atggaagcgt tcaactagca 540

gaccattatc aacaaaatac tccaattggc gatggccctg tccttttacc agacaaccat 600

tacctgtcca cacaatctgc cctttcgaaa gatcccaacg aaaagagaga ccacatggtc 660

cttcttgagt ttgtaacagc tgctgggatt acacatggca tggatgaact gtacaactga 720

<210> 2

<211> 25

<212> DNA

<213> Artificial Sequence

<220>

<223> Description of Artificial Sequence: PCR primer LTR I to amplify a

DNA fragment having LTR sequence.

<400> 2

agctgttcca tctgttcttg gccct                                        25

<210> 3

<211> 38

<212> DNA
<213> Artificial Sequence

<220>
<223> Description of Artificial Sequence: Oligonucleotide constituting
linker-cassette A.

<400> 3
gacccgggag atctgaattc agtggcacag cagttagg                              38

<210> 4
<211> 42
<212> DNA
<213> Artificial Sequence

<220>
<223> Description of Artificial Sequence: Oligonucleotide constituting
linker-cassette A.

<400> 4
aattcctaac tgctgtgcca ctgaattcag atctcccggg tc                         42

<210> 5
<211> 20
<212> DNA
<213> Artificial Sequence

<220>
<223> Description of Artificial Sequence: PCR primer LTR II to amplify

a DNA fragment having LTR sequence.

<400> 5
gaccttgatc tgaacttctc                                                    20

<210> 6
<211> 20
<212> DNA
<213> Artificial Sequence

<220>
<223> Description of Artificial Sequence: PCR primer LC1 to amplify a
DNA fragment having linker-cassette A.

<400> 6
gacccgggag atctgaattc                                                    20

<210> 7
<211> 20
<212> DNA
<213> Artificial Sequence

<220>
<223> Description of Artificial Sequence: PCR primer LTR III to amplify
a DNA fragment having LTR sequence.

<400> 7
tccatgcctt gcaaaatggc                                                    20

<210> 8

<211> 21

<212> DNA

<213> Artificial Sequence

<220>

<223> Description of Artificial Sequence: PCR primer LC2 to amplify a

DNA fragment having linker-cassette A.

<400> 8

gatctgaatt cagtggcaca g                                                21

<210> 9

<211> 38

<212> DNA

<213> Artificial Sequence

<220>

<223> Description of Artificial Sequence: Oligonucleotide constituting

linker-cassette B.

<400> 9

gacccgggag atctgaattc agtggcacag cagttagg                              38

<210> 10

<211> 38

<212> DNA

<213> Artificial Sequence

<220>

<223> Description of Artificial Sequence: Oligonucleotide constituting

linker-cassette B.

<400> 10

cctaactgct gtgccactga attcagatct cccgggtc 38

## Description

### Technical Field

The present invention relates to a method for analyzing a gene integration site on a chromosome in a cell having a transferred gene.

### Background Art

Gene therapy is therapy in which intractable diseases such as genetic diseases or cancers which are due to "errors in genetic information" in cells are treated or prevented, for example, by providing correct genetic information to repair the functions of the cells, or by adding a new "protective gene" which the cells do not have originally.

Known methods used in gene therapy for transferring genes into cells include methods in which virus vectors are used, method in which naked DNAs are transferred by means of endocytosis, electroporation or gene gun, and methods in which gene transferring agents such as liposome are used.

Virus vectors are utilized in the field of gene therapy for a wide variety of purposes including basic and clinical purposes. For example, adenovirus vectors are suitable for transiently expressing a gene of interest in target cells abundantly. On the other hand, it is expected that retrovirus vectors are utilized for gene therapy for genetic diseases or in the field of transgenic animal production because the retrovirus vectors have a function of stably integrating a gene of interest into a host chromosome and enable long-term stable expression of the gene. However, since a gene of interest is integrated into a host chromosome at random according to a method for transferring a gene using a retrovirus vector, possibility of canceration of a cell depending on the integration site has been pointed out. Thus, analysis of the site of retrovirus vector-mediated gene integration on the host chromosome and sensitive monitoring for determining the clone species (monoclonal, oligoclonal, polyclonal) of a test cell population have become important.

The inverse PCR method (see J. Virol., 63:1924-1928 (1989)) or the LM PCR method (see Science, 246:780-786 (1989)) is generally used as a method for analyzing a site of insertion or integration of a virus, a transposon, a transgene or the like into a chromosomal DNA. However, it has been pointed out that such a method has problems such as low detection sensitivity (requirement of much template DNA) or low specificity.

Then, the linear amplification mediated PCR (LAM PCR) method was developed in order to overcome the problems (see WO 00/24929; Blood, 100:2737-2743 (2002)). A linear PCR using a chromosomal DNA as a template and a primer is first conducted according to this method. The primer is complementary to a long terminal repeat (LTR) sequence, which is a sequence characteristic of a retrovirus, and labeled with biotin at its end. Only one primer is used in the linear PCR to amplify a single-stranded DNA corresponding to a portion downstream of the position to which the primer anneals. The product of the linear PCR is adsorbed to magnetic beads having immobilized streptavidin to efficiently recover a single-stranded amplified DNA fragment having the LTR sequence and a sequence derived from the chromosome. The single-stranded DNA is converted into a double-stranded DNA by synthesizing the complementary strand. The double-stranded DNA is cleaved with a restriction enzyme that recognizes a four base sequence and cleaves the double-stranded DNA at the sequence. A double-stranded DNA called a linker cassette is ligated to the terminus. A PCR is conducted using the thus obtained ligation product as a template as well as a primer complementary to the LTR and a primer complementary to the linker cassette. A DNA fragment that contains the LTR and a chromosome-derived DNA flanking the LTR is then amplified. Thus, one can analyze the retrovirus integration site. Furthermore, it is possible to analyze the site of integration in the host chromosomal DNA by recovering the PCR-amplified fragment using agarose gel or the like, subcloning it into an appropriate vector, and determining the nucleotide sequence of the fragment.

The detection sensitivity is improved according to this method as compared with conventional methods. In addition, the method is advantageous because the use of magnetic beads simplifies the purification of the sample between reaction steps and decreases the amount of the chromosomal DNA as a template.

However, use of the current reaction conditions may lead to a phenomenon of biased amplification of fragment(s) from specific clone(s) depending on the kind or amount of the DNA as a template if a chromosomal DNA obtained from a biological sample-derived population having many different integration sites (polyclone) is to be analyzed. In this case, the results of amplification do not reflect the actual state of clones existing in the cell population in the used sample. Furthermore, a low-abundance clone in the sample may not be detected. Thus, the conventional LAM PCR method has problems concerning its sensitivity, reproducibility and accuracy. Therefore, it is difficult to obtain information that reflects the actual state of a gene-transferred cell population and form a correct judgment.

As described above, the LAM PCR method is currently considered to be an effective system for analyzing a gene integrated using a retrovirus vector. However, the method needs to be improved in order to accurately, sensitively and reproducibly monitor a biological sample having a retrovirus integrated at various sites for the extent of cells having an integrated gene in the population (polyclonal, oligoclonal or monoclonal), or the ratio of a specific cell in the population.

### Disclosure of Invention

The main object of the present invention is to improve the conventional method for analyzing an integration sites using the LAM PCR method to construct a system in which more integration fragments are amplified without being biased toward a fragment amplified from a specific clone by a PCR reaction, for example, using a population of cells having various retrovirus-mediated gene integration sites, and by which a specific amplified fragment contained in the population is sensitively detected. Thereby, it is possible to judge the clone species (monoclonal, oligoclonal, polyclonal) of the test cell population and monitor the content of a specific cell in the cell population with high sensitivity and reproducibility.

As a result of intensive studies for achieving the above-mentioned object, the present inventors have found that sensitive detection of nonbiased amplified fragments is made possible by conducting a primer extension reaction in the presence of a compound that lowers a Tm value of a double-stranded nucleic acid to improve the reaction scheme including PCR conditions. Thus, the present invention has been completed.

The first aspect of the present invention relates to a method for analyzing a region of a chromosome-derived DNA flanking a retrovirus vector in a chromosomal DNA having the retrovirus vector being integrated, the method comprising:
(1) conducting a primer extension reaction using a primer that has a sequence complementary to a nucleotide sequence of an LTR of a retrovirus vector, and a chromosomal DNA having the retrovirus vector being integrated as a template to obtain a primer extension product;
(2) recovering the primer extension product obtained in step (1) and synthesizing a DNA that is complementary to the primer extension product to obtain a double-stranded DNA;
(3) adding a double-stranded oligonucleotide to the terminus of the double-stranded DNA obtained in step (2); and
(4) conducting a nucleic acid amplification reaction using the double-stranded DNA having the added oligonucleotide obtained in step (3) as a template, as well as a primer that has a sequence complementary to the nucleotide sequence of the LTR of the retrovirus vector and a primer that has a sequence complementary to the nucleotide sequence of the double-stranded oligonucleotide to obtain an amplification product,
wherein the primer extension reaction in step (1) and the nucleic acid amplification reaction in step (4) are conducted in the presence of a compound that lowers a Tm value of a double-stranded nucleic acid.

According to the method of the first aspect, the primer extension reaction in step (1) and/or the nucleic acid amplification reaction in step (4) may be conducted using a polymerase chain reaction. A DNA polymerase composition which is a mixture of a DNA polymerase that has a 3' exonuclease activity and a DNA polymerase that does not have a 3' exonuclease activity may be used for the polymerase chain reaction. For example, the temperature cycle of the polymerase chain reaction consists of 95°C for 60 seconds; 58°C for 45 seconds; and 72°C for 90 seconds.

The compound that lowers a Tm value of a double-stranded nucleic acid used in the method of the first aspect is exemplified by a substance selected from the group consisting of betaines, formamide, dimethyl sulfoxide and tetramethylammonium salts. For example, trimethylglycine may be used as a betaine.

A two-step polymerase chain reaction may be used in step (4) of the first aspect.

In step (3) of the first aspect, the double-stranded DNA obtained in step (2) may be digested with a restriction enzyme and the double-stranded oligonucleotide may be added to the generated terminus of the double-stranded DNA. The chromosomal DNA in step (1) may have been digested with a restriction enzyme beforehand.

According to the first aspect, a primer having a label may be used in step (1) to recover the primer extension product utilizing the label in step (2). The label is exemplified by biotin.

The method of the first aspect may further comprise a step of determining a nucleotide sequence of the amplification product obtained in step (4). In the method, the nucleotide sequence may be determined using, as a template, a recombinant DNA in which the product obtained in step (4) is incorporated into a vector.

The second aspect of the present invention relates to a kit for determining a nucleotide sequence of a chromosome-derived DNA flanking a retrovirus vector in a chromosomal DNA having the retrovirus vector being integrated, the kit containing:
(a) a primer that is capable of annealing to an LTR portion of a retrovirus;
(b) a linker cassette;
(c) a primer that is capable of annealing to the linker cassette of (b);
(d) a DNA polymerase;
(e) a restriction enzyme; and
(f) a buffer for a primer extension reaction which contains a compound that lowers a Tm value of a double-stranded nucleic acid.

The DNA polymerase contained in the kit of the second aspect may be a DNA polymerase composition which is a mixture of a DNA polymerase that has a 3' exonuclease activity and a DNA polymerase that does not have a 3' exonuclease activity. The compound that lowers a Tm value of a double-stranded nucleic acid is exemplified by a substance selected from the group consisting of betaines, formamide, dimethyl sulfoxide (DMSO) and tetramethylammonium salts. Trimethylglycine may be used as a betaine.

The primer contained in the kit of the second aspect may have a label for recovering a primer extension product.

The third aspect of the present invention relates to a buffer for a primer extension reaction used for a method for analyzing a region of a chromosome-derived DNA flanking a retrovirus vector in a chromosomal DNA having the retrovirus vector being integrated, which contains a compound that lowers a Tm value of a double-stranded nucleic acid.

The fourth aspect of the present invention relates to use of a compound that lowers a Tm value of a double-stranded nucleic acid for the manufacture of a buffer for a primer extension reaction used for a method for analyzing a region of a chromosome-derived DNA flanking a retrovirus vector in a chromosomal DNA having the retrovirus vector being integrated.

### Best Mode for Carrying out the Invention

### 1. The method for analyzing a gene integration site of the present invention

### (1) Synthesis of DNA (primer extension product) containing chromosomal DNA flanking LTR

A primer used for a primer extension reaction using a chromosomal DNA as a template according to the present invention has a sequence complementary to a nucleotide sequence of an LTR of a retrovirus vector. There is no specific limitation concerning the primer as long as it is capable of annealing to an LTR portion under conditions under which a primer extension reaction with a DNA polymerase takes place. It can be designed and prepared on the basis of the nucleotide sequence of the LTR at will. The primer may contain a nucleotide that is not complementary to the LTR sequence as long as it is capable of annealing to the LTR portion. Although the length is not specifically limited, for example, a primer having a chain length of 12-50 nucleotides, preferably 15-40 nucleotides may be used.

There is no specific limitation concerning the retrovirus vector to which the method of the present invention can be applied. If the LTR sequence of the retrovirus vector is known, a primer can be prepared on the basis of the sequence and the method of the present invention can be carried out. The primer used according to the present invention is exemplified by a primer having the nucleotide sequence of SEQ ID NO:2.

The primer preferably has a label for facilitating recovery of a primer extension product. A primer labeled with a ligand for which a specific receptor is known (e.g., biotin), a hapten or the like can be used.

Although there is no specific limitation concerning the chromosomal DNA used as a sample according to the present invention, it is usually a chromosomal DNA prepared from a cell into which a gene has (or may have) been transferred using a retrovirus vector. The cell that serves as a material for preparing the chromosomal DNA may be derived from a monoclonal, oligoclonal or polyclonal population. There is also no specific limitation concerning the cell. It may be a cell collected from a living body or a cell cultured ex vivo. In case of a cell collected from a living body, if a hematopoietic cell such as a hematopoietic stem cell is a target for gene transfer, the cell, a cell differentiated from the cell (e.g., T cell, B cell, monocyte, macrophage, etc.) or a mixture thereof may be used as a material for preparing the chromosomal DNA to be used according to the method of the present invention.

The primer is annealed to a chromosomal DNA having a retrovirus vector being integrated, and a DNA extension reaction from the primer is conducted to synthesize a DNA strand containing an LTR and a chromosomal DNA flanking the LTR. A DNA polymerase is used for this step.

There is no specific limitation concerning the DNA polymerase used according to the present invention as long as it can be used to synthesize a DNA strand complementary to a DNA strand as a template. For example, a DNA polymerase derived from *Escherichia coli,* a DNA polymerase derived from a thermophilic bacterium of the genus *Bacillus* (Bst DNA polymerase, Bca DNA polymerase, etc.), a DNA polymerase derived from a bacterium of the genus *Thermus* (Taq DNA polymerase, Tth DNA polymerase, etc.), a DNA polymerase derived from an archaebacterium (Pfu DNA polymerase, Vent DNA polymerase, Deep Vent DNA polymerase, KOD DNA polymerase, etc.) or a variant thereof can be used according to the present invention.

Furthermore, a mixture of two or more enzymes may be used. For example, a method in which a mixture of a DNA polymerase that has a 3' exonuclease activity (e.g., Pfu DNA polymerase, Vent DNA polymerase, Deep Vent DNA polymerase, KOD DNA polymerase, etc.) and a DNA polymerase that does not have a 3' exonuclease activity (e.g., Taq DNA polymerase, Tth DNA polymerase, an exonuclease-deficient mutant of the DNA polymerase that has a 3' exonuclease activity) is used is particularly preferable according to the present invention. Such a method is disclosed, for example, in United States Patent No. 5436149. Furthermore, TaKaRa Ex Taq, TaKaRa LA Taq (both from Takara Bio) or the like is commercially available as a DNA polymerase composition which is a mixture of a DNA polymerase that has a 3' exonuclease activity and a DNA polymerase that does not have a 3' exonuclease activity.

For obtaining a much primer extension product, it is desirable to repeat a primer extension reaction several times. In this case, synthesis of a primer extension product may be conducted using a PCR in which the following three steps are repeated: dissociation of an extension product from a DNA as a template; annealing of a new primer to the DNA as a template; and primer extension reaction.

According to the conventional LAM PCR method, the step of obtaining a primer extension product is carried out using a polymerase chain reaction (PCR). Depending on the kind or the amount of the chromosomal DNA as a template, a primer extension product in which a specific molecule is enriched is obtained using a PCR in many cases. Then, one may fail to obtain a primer extension product rich in variety.

The present inventors have shown that it is possible to obtain a primer extension product composed of more molecular species than the conventional LAM PCR method by conducting a primer extension reaction under altered PCR reaction conditions using a reaction mixture containing a compound that lowers a Tm value of a double-stranded nucleic acid. Examples of such compounds include betaines (e.g., trimethylglycine), organic solvents (e.g., formamide, dimethyl sulfoxide) and tetramethylammonium salts (tetramethylammonium chloride (TMAC), tetramethylammonium acetate (TMAA), tetramethylammonium hydroxide (TMAH)). A mixture of a DNA polymerase that has a 3' exonuclease activity and a DNA polymerase that does not have a 3' exonuclease activity is preferably used as a DNA polymerase for PCR.

A PCR reaction may be carried out under conditions determined based on description of a known literature or the like. Among the above-mentioned polymerases, a polymerase that is highly heat-resistant (e.g., a DNA polymerase derived from a bacterium of the genus *Thermus* or a DNA polymerase derived from an archaebacterium) or a mixture thereof can be used as a DNA polymerase. A composition of a reaction mixture suitable for a DNA polymerase to be used may be selected, and the above-mentioned compound that lowers a Tm value of a double-stranded nucleic acid may be added thereto. Two or more of such compounds may be added in combination. There is no specific limitation concerning the concentration as long as the desired effect is achieved. For example, a betaine at a final concentration of 0.1-3 M, preferably 0.1-1 M, dimethyl sulfoxide at a final concentration of 0.5-15%, preferably 1-10%, or a tetramethylammonium salt at a final concentration of 0.1-100 mM, preferably 1-50 mM can be used according to the present invention.

Also, there is no specific limitation concerning the PCR reaction cycle. An exemplary cycle consists of 95°C for 60 seconds; 58°C for 45 seconds; and 72°C for 90 seconds. The temperature may vary in the range of ±2°C, and/or the time may vary in the range of ±30%. The cycle number may be determined taking the amplification efficiency or the like into consideration. For example, 20-150 cycles, preferably 40-100 cycles of the reaction may be conducted.

A suitable amount of a primer used in a primer extension reaction is 0.025-1.0 pmol for 100 ng of a chromosomal DNA or in 50 µl of a reaction mixture.

### (2) Conversion of primer extension product into double strands

A primer extension product synthesized in the step as described above is recovered from the reaction mixture, for example, utilizing a label added to a primer.

If a ligand as a label is added to a primer, a receptor that binds to the ligand can be used. In case of labeling with a hapten, an antibody that recognizes the hapten can be used. A primer extension product can be readily recovered from a reaction mixture by utilizing a solid phase onto which a substance that binds to such a labeling substance is immobilized. For example, a primer extension product obtained using a primer labeled with biotin can be separated from a chromosomal DNA as a template as follows. Beads having immobilized avidin are added to an extension reaction mixture to capture the extension products on the beads. The beads are washed, and the extension products are then detached from the beads.

Furthermore, unreacted primers may be removed before recovering a primer extension product. For example, the removal can be accomplished by filtration using a filter having an appropriate pore size or a fractional precipitation method under conditions under which only long nucleic acids are selectively precipitated.

The recovered primer extension product in a form of a single-stranded DNA is then converted into a double-stranded DNA. There is no specific limitation concerning the means of conversion. For example, a double-stranded nucleic acid can be obtained by annealing a random primer to the primer extension product, and synthesizing a DNA complementary to the primer extension product from the random primer using a DNA polymerase.

### (3) Addition of linker cassette

The primer extension product which has been converted into double strands in step (2) above is digested with a restriction enzyme, and a double-stranded oligonucleotide is added to a terminus generated as a result of the digestion.

Although there is no specific limitation concerning the restriction enzyme used according to the present invention, it is inappropriate to use one whose recognition/cleavage site is present in a nucleotide sequence of an LTR portion contained in a primer extension product. A restriction enzyme that results in a cohesive end is preferable according to the present invention for efficiency of subsequent addition of a double-stranded oligonucleotide. Furthermore, a restriction enzyme that recognizes a four base sequence is preferable for obtaining DNA fragments of appropriate lengths. For example, Tsp509I (Sse9I), MboI (Sau3AI) or the like can be used. Digestion with a restriction enzyme is conducted under conditions suitable for the restriction enzyme to be used.

A double-stranded oligonucleotide (linker cassette) is added to a terminus of a double-stranded primer extension product digested with a restriction enzyme. The linker cassette has a terminus that corresponds to the terminus generated as a result of the restriction enzyme digestion (preferably a cohesive end), and a sequence to which a primer used in a subsequence step of nucleic acid amplification is capable of annealing. There is no specific limitation concerning the sequence or the chain length thereof as long as the linker cassette meets the above-mentioned requirements. Usually, a linker cassette of 20-100 base pairs, preferably 30-60 base pairs is used according to the present invention. There is also no specific limitation concerning the sequence to which a primer is capable of annealing. Any sequence can be used except a sequence that is inappropriate for design of a primer (biased GC content, possibility of formation of intramolecular or intermolecular hydrogen bonds).

A linker cassette can be added using a known means of connecting DNA (a ligation reaction). For example, a commercially available kit or the like may be used.

### (4) Amplification of double-stranded DNA

The thus obtained double-stranded DNA has an LTR-derived nucleotide sequence at one end and a nucleotide sequence of a linker cassette at another end. The double-stranded DNA can be amplified by conducting a nucleic acid amplification reaction using this DNA as a template, as well as a primer that is capable of annealing to the LTR-derived nucleotide sequence and a primer that is capable of annealing to the nucleotide sequence of the linker cassette.

There is no specific limitation concerning the nucleic acid amplification method used for the method of the present invention. A known nucleic acid amplification method such as the PCR method, the ICAN method, the SDA method or the TMA method can be used.

If the PCR method is to be used, suitable conditions (composition of the reaction mixture, cycling conditions) may be determined according to known methods. Preferably, a reaction mixture containing a compound that lowers a Tm value of a double-stranded nucleic acid and a mixture of a DNA polymerase that has a 3' exonuclease activity and a DNA polymerase that does not have a 3' exonuclease activity are used according to the method described in (1) above. Regarding the cycling conditions, although those as described in (1) above may be used, a less cycle number may be used. A two-step PCR method known as the nested PCR method may be used.

A DNA fragment containing a nucleotide sequence of an LTR portion of a retrovirus vector and a nucleotide sequence of a region of a chromosome flanking the site at which the vector is integrated (i.e., flanking the LTR) can be obtained by the series of steps (1) to (4).

Alternatively, a chromosomal DNA used in step (1) as a template may be digested beforehand with an appropriate restriction enzyme such as the one as described in (3), and a primer extension reaction may be conducted using the digested chromosomal DNA. In this case, the resulting extension product is converted into a double-stranded DNA and a linker cassette is added thereto without subjecting it to digestion with a restriction enzyme. A linker cassette having a blunt end is used for this purpose.

It is possible to examine the degree of variation of integration sites in a cell population used as a sample by analyzing the amplified DNA fragment obtained according to the method of the present invention, for example, using agarose gel electrophoresis.

Furthermore, the nucleotide sequence of the amplified DNA fragment can be determined according to a known method (e.g., the dideoxy method).

The amplified DNA fragment is recovered from the reaction mixture and then ligated to an appropriate vector (a plasmid vector, a phage vector, etc.). The recombinant DNA molecule is used to transform an appropriate host. The recombinant DNA molecule is prepared from the resulting transformant. A sequencing reaction is carried out using the recombinant DNA molecule as a template to determine the nucleotide sequence of the DNA fragment. Although it is not intended to limit the present invention, a vector having a T nucleotide protruding at its 3' end (T-vector) is preferable for cloning a DNA fragment amplified using the PCR method.

If the molecular species of the amplified DNA fragment is limited and the DNA fragment can be isolated using agarose gel electrophoresis or the like, direct sequencing may be carried out using the isolated amplified DNA fragment as a template.

A sequence flanking a retrovirus vector-derived sequence in a chromosomal DNA as a starting material is contained in the thus obtained nucleotide sequence information. Thus, one can obtain information on the site on the chromosome at which the retrovirus vector is integrated by comparing the sequence with a known nucleotide sequence of chromosomal DNA.

Even if a chromosomal DNA derived from a polyclone is used as a sample, a library consisting of DNA fragments having various sequences in which a DNA fragment having a specific sequence is not enriched can be obtained according to the method of the present invention. By analyzing sequences of DNA fragments in such a library, it is possible, for example, to determine a site on a chromosome at which a retrovirus vector is integrated, to estimate a sequence or a region whose integration efficiency is high, and to monitor a distribution or a ratio of a specific gene-transferred cell in a living body.

Since the sensitivity of the method of the present invention is excellent as compared with the conventional LAM PCR method, good results can be obtained with reproducibility even using a chromosomal DNA with a relatively low rate of gene transfer.

### 2. Kit used for the analysis method of the present invention

A kit used for the gene integration site analysis method of the present invention contains reagents used in the respective steps as described in 1 above. For example, it contains the following components:
(a) a primer that is capable of annealing to an LTR portion of a retrovirus;
(b) a linker cassette;
(c) a primer that is capable of annealing to the linker cassette of (b);
(d) a DNA polymerase;
(e) a restriction enzyme; and
(f) a buffer for primer extension which contains a compound that lowers a Tm value of a double-stranded nucleic acid.

Among the components, the buffer of (f) contains a compound that lowers a Tm value of a double-stranded nucleic acid. Examples of such compounds are described above. It is preferable that the composition thereof is suitable for use of the DNA polymerase of (d). The kit of the present invention includes one in a form, for example, in which the primer and the DNA polymerase have been added to the buffer beforehand, or dNTPs and/or an Mg salt is attached being separated from the buffer.

If the primer extension reaction is conducted using PCR, a heat-resistant one is selected as the DNA polymerase of (d). A DNA polymerase composition which is a mixture of a DNA polymerase that has a 3' exonuclease activity and a DNA polymerase that does not have a 3' exonuclease activity may be contained as the DNA polymerase.

A label suitable for recovery of an extension product such as biotin may be added to the primer of (a). In this case, it is preferable that the kit further contains a component for purification of the extension product corresponding to the label such as beads having immobilized avidin.

The kit of the present invention may contain a component other than the above-mentioned components such as a reagent for conversion of a primer extension product into double strands, a buffer for restriction enzyme reaction or a reagent for linker cassette ligation.

Using the kit, a retrovirus integration site can be readily analyzed according to the present invention.

If a chromosomal DNA obtained from a population having many different integration sites (polyclone) is to be analyzed, fragment(s) biasedly amplified from specific clone(s) may be obtained depending on the kind or the amount of the DNA as a template. This phenomenon has been a problem associated with the retrovirus integration site analysis method using the conventional LAM PCR method. The phenomenon is overcome according to the present invention. Thus, according to the method of the present invention, it is possible to monitor the extent of cells having an integrated gene using a retrovirus vector in a population (polyclonal, oligoclonal or monoclonal), or the ratio of a specific cell in a population with accuracy, high sensitivity and high reproducibility.

A method for analyzing a retrovirus integration site using the conventional LAM PCR method tends to result in PCR amplification product(s) biasedly derived from specific clone(s) depending on the kind or the amount of the genomic DNA used as a template. Then, it has been considered that the number of fragments corresponding to the number of integration sites may not be observed, and problems such as low reproducibility may become obstacles to accurate judgment. However, such problems are solved according to the method of the present invention, making highly precise judgment possible.

### Examples

The following Examples illustrate the present invention in more detail, but are not to be construed to limit the scope thereof.

### Example 1

Analysis of 293 cells and CD34-positive cells having integrated retrovirus (analysis of polyclones)

### (1) Preparation of retrovirus supernatant

293 cells (ATCC CRL-1573) or PG13 cells (ATCC CRL-10686) were cultured in Dulbecco's modified Eagle medium (DMEM, Sigma) containing 10% fetal bovine serum (JRH), 50 µg/ml of penicillin (Gibco) and 50 µg/ml of streptomycin (Gibco) at 37°C in the presence of 5% CO₂. DMEM used in procedures below contains 50 µg/ml of penicillin and 50 µg/ml of streptomycin in all cases.

An amphotropic retrovirus vector was prepared as follows. Briefly, a gene encoding red-shift green fluorescent protein (hereinafter referred to as GFP) inserted in a plasmid pQBI25 (Quantum Biotechnologies Inc.) (SEQ ID NO:1) was inserted into a plasmid pDON-AI (Takara Bio) to construct pDON-GFP.

The plasmid pDON-GFP was transferred into 293. cells using Retrovirus Packaging Kit Ampho (Takara Bio), and a culture supernatant was collected according to the instructions attached to the kit. The culture supernatant was filtered through a 0.45-micron filter (Millipore) to prepare a stock of amphoGFP virus supernatant, which was stored at -80°C until use. The infectivity titer of the amphoGFP virus supernatant against NIH/3T3 cells was 4 x 10⁶ infective viral particles/ml.

GALV pseudo-type retrovirus vector was prepared as follows. Briefly, two rounds of gene transfer into 2 x 10⁴ of PG13 cells were carried out according to the RetroNectin method (J. Biochem., 130:331-334 (2001)) using 200 µl of the stock of amphoGFP virus supernatant prepared previously. The cells were selectively cultured for two weeks in DMEM medium containing 500 µg/ml of G418 and 10% fetal bovine serum. The resulting cells were used as GALV pseudo-type retrovirus-producer cells. The producer cells were grown to semi-confluence in a 10-cm dish. The medium was then exchanged for 7 ml of fresh DMEM containing 10% FBS. Cultivation was further continued for 24 hours at 37°C in the presence of 5% CO₂. The supernatant was filtered through a 0.45-micron filter (Millipore) to prepare a stock of galvGFP virus supernatant, which was stored at -80°C until use. The infectivity titer of the galvGFP virus supernatant against HT1080 cells was 6 x 10⁵ infective viral particles/ml.

### (2) Transfer of retrovirus vector into 293 cells and CD34-positive cells

1 x 10⁷ of 293 cells mixed with 2 ml of the galvGFP virus supernatant prepared in Example 1(1) were seeded into two 10-cm RetroNectin-coated plates (Takara Bio) for viral infection. The cells were cultured for 24 hours at 37°C in the presence of 5% CO₂, detached using trypsin, dispensed into six 10-cm tissue culture plates, and further cultured for 48 hours at 37°C in the presence of 5% CO₂. Then, cells were collected by trypsinization, transferred into a centrifuge tube, washed twice with PBS, and stored at ―80°C. The group of transferred cells was designated as 293R1. Analysis of 293R1 using a fluorescence-activated cell sorter (FACS) revealed a gene transfer efficiency of 59%.

Gene transfer into human CD34-positive hematopoietic stem cells was carried out as follows. CD34-positive cells (BioWhittaker), which had been cytokine-stimulated for 24 hours, mixed with 1 ml of the amphoGFP virus supernatant were added to 6-cm RetroNectin-coated plates (Takara Bio) for the first viral infection. The total cell number upon infection was 1.14 x 10⁶. The cells were cultured for 24 hours at 37°C in the presence of 5% CO₂. A supernatant was removed by centrifugation. After adding thereto 2 ml of a cytokine medium (300 ng/ml of stem cell factor (Genzyme), 100 ng/ml of thrombopoietin (PeproTech House), 60 ng/ml of human interleukin-3 (Genzyme), 300 ng/ml of human flt-3/flk-2 ligand (R&D Systems) and 4% fetal bovine serum (BioWhittaker)) and 1 ml of the amphoGFP virus supernatant, the cells were cultured for 24 hours at 37°C in the presence of 5% CO₂. A supernatant was removed by centrifugation. After adding thereto 2 ml of the cytokine medium and 1 ml of the amphoGFP virus supernatant, the cells were cultured for 24 hours at 37°C in the presence of 5% CO₂. After cultivation, cells were collected by trypsinization, and stored at -80°C. The group of transferred cells was designated as CD+R1. Analysis of CD+R1 using a FACS revealed a gene transfer efficiency of 20.3%.

### (3) Preparation of chromosomal DNA

Frozen cells (293R1 or CD+R1, corresponding to about 2.0 x 10⁶ cells) stored at -80°C were suspended in 5 ml of a suspension solution (10 mM tris-hydrochloride buffer (pH 8.0), 10 mM EDTA, 150. mM sodium chloride). 1 µl of an RNase A solution (10 mg/ml), 50 µl of a 10% SDS solution, and 25 µl of proteinase K (20 mg/ml, Takara Bio) were added thereto. The mixture was mixed by inversion and heated at 50°C for 3 hours. An equal volume of a phenol solution was added thereto, and the mixture was mixed by inversion for 15 minutes. A supernatant was collected by centrifugation at room temperature, an equal volume of a solution of phenol : chloroform : isoamylalcohol was added thereto, and the mixture was mixed by inversion for 15 minutes. 1/25 volume of 5 M sodium chloride was added to and mixed with a supernatant collected by centrifugation. 2.5 volumes of ethanol were further added thereto. The mixture was allowed to stand at room temperature, and a resulting precipitate was collected. The precipitate was washed in 70% ethanol, and air-dried for 10 minutes. TE buffer (10 mM tris-hydrochloride buffer (pH 8.0), 1 mM EDTA) was added thereto, and the precipitate was dissolved by allowing to stand in a cold room for 24 hours. Chromosomal DNA samples were obtained from 293R1 and CD+R1 as a result of the above-mentioned procedure.

### (4) Analysis of gene integration site

25 µl of x2 PCR buffer (100 mM tris-hydrochloride buffer (pH 9.2), 28 mM ammonium sulfate, 20 mM potassium chloride, 5.0 mM magnesium chloride, 0.02%(w/v) BSA, 10%(v/v) DMSO, 0.5 M betaine (trimethylglycine)), 5 µl of dNTP mix (2.5 mM each), 0.1 pmol of a primer LTR I which has biotin added at its 5' end (LTR I has a sequence complementary to the LTR of the retrovirus vector; SEQ ID NO:2), 0.25 µl of Ex Taq (5 U/µl, Takara Bio) and sterile water to a volume of 50 µl were added to 100 ng of the genomic DNA prepared from 293R1 or CD34+R1 in Example 1-(3). The mixture was placed in an automated gene amplification apparatus thermal cycler (Takara Bio) and subjected to a PCR as follows: denaturation at 95°C for 5 minutes; 50 cycles of 95°C for 60 seconds (denaturation), 58°C for 45 seconds (primer annealing) and 72°C for 90 seconds (synthesis reaction)); and incubation at 72°C for 10 minutes.

After the reaction, unreacted primers were removed as follows. 300 µl of TE solution was added to 50 µl of the PCR reaction mixture. The mixture was added to Suprec02 (Takara Bio) and centrifuged at 5,000 rpm for about 7 minutes. 350 µl of TE solution was further added thereto, and centrifugation was carried out in a similar manner to wash the filter. TE solution was added to the filter to a volume of 40 µl to collect a DNA solution from which primers had been removed.

Magnetic beads (40 µl, corresponding to 200 µg) having immobilized streptavidin (MPG streptavidin, Takara Bio) were added to the solution. The mixture was allowed to stand for 1 hour at room temperature while occasionally mixing. A tube containing the reaction mixture was placed for 1 minute on a magnetic stand (Magnetight Separation Stand, Takara Bio). Then, a supernatant was discarded to collect the beads. The beads were gently mixed with 100 µl of BW buffer (5 mM tris-hydrochloride buffer (pH 7.5), 0.5 mM EDTA, 1.0 M sodium chloride). After standing on the magnetic stand, a supernatant was discarded in a similar manner. A similar procedure was carried out with 100 µl of sterile water to collect the beads (this procedure is called a beads washing procedure).

The washed beads were gently suspended in 14.1 µl of sterile water. 2 µl of x10 Klenow buffer (attached to Klenow fragment below), 2.4 µl of dNTP mix (2.5 mM each), 1 µl of a random primer (random 6mer, 100 pmol/µl, Takara Bio) and 0.5 µl of Klenow fragment (Takara Bio) were added to the beads to prepare 20 µl of a mixture. The mixture was reacted at 37°C for 1 hour while occasionally mixing to synthesize a complementary strand. After reaction, the beads were washed. The collected beads were suspended in 17.5 µl of sterile water. 2 µl of x10 NE buffer 1 (attached to Tsp509I below) and 0.5 µl of a restriction enzyme Tsp509I (10 U/µl, New England Biolabs) were added thereto. The mixture was incubated at 65°C for 1 hour while occasionally mixing. After the beads washing procedure, 2 µl of linker cassette A (50 pmol/µl) (nucleotide sequences of two oligonucleotides constituting the linker cassette A are shown in SEQ ID NOS:3 and 4), 16 µl of Solution A (Takara Ligation kit ver. 1, Takara Bio) and 2 µl of Solution B (Takara Ligation kit ver. 1, Takara Bio) were added to the beads. The mixture was reacted at 16 °C for 1 hour while occasionally mixing.

After washing the beads once with sterile water, the beads were collected, suspended in 5 µl of 0.1 N sodium hydroxide, and allowed to stand at room temperature for 10 minutes. A supernatant (about 5 µl) was collected on the magnetic stand, and 1 µl of the supernatant was used for a PCR reaction as follows. 25 µl of x2 PCR buffer, 5 µl of dNTP mix (2.5 mM each), 1 µl each of a primer LTR II which has a sequence complementary to the LTR (SEQ ID NO:5, 25 pmol/µl) and a primer LC1 which has a sequence complementary to the linker cassette (SEQ ID NO: 6, 25 pmol/µl), 0.25 µl of Ex Taq and sterile water to a volume of 50 µl were added to 1 µl of the solution. The reaction mixture was placed in an automated gene amplification apparatus thermal cycler and subjected to a PCR as follows: denaturation at 95°C for 5 minutes; 30 cycles of 95°C for 60 seconds (denaturation), 58°C for 45 seconds (primer annealing) and 72°C for 90 seconds (synthesis reaction); and incubation at 72°C for 10 minutes (the first round). Then, the second round of PCR was carried out using 1 µl of the reaction mixture of the first round of PCR as a template as well as a primer LTR III (SEQ ID NO:7, 25 pmol/µl) and a primer LC2 (SEQ ID NO:8, 25 pmol/µl). The primer LTR III has a sequence complementary to a portion in the LTR downstream of the sequence to which LTRII anneals, and the primer LC2 has a sequence complementary a portion in the linker cassette downstream of the sequence to which LC1 anneals. The conditions used for the second round of PCR were the same as those used for the first round of PCR.

The amplification product in the thus obtained reaction mixture was confirmed by subjecting 5 µl of the reaction mixture (50 µl) to electrophoresis on 3.0%(w/v) agarose gel. As a result, it was confirmed that various amplified fragments of about 500 bp or less were run forming a broad band in case where the chromosomal DNA derived from either 293R1 or CD+R1 was used as a template. These results reflect the fact that the chromosomal DNA used as a template was prepared from a polyclonal population consisting of cells in which the retrovirus vector had been integrated at various positions on the chromosome. Furthermore, it was shown that amplification not biased toward a fragment derived from a specific clone is possible according to the above-mentioned method.

The fragments amplified using the genomic DNA derived from CD34+R1 as a template were recovered from 3.0% low melting point agarose gel and ligated to pT7BlueT vector (Takara Bio). The ligation product was used to transform *Escherichia coli* JM109. Sixty-eight clone candidate strains each harboring the vector with an inserted fragment being ligated were selected from white colonies grown on solid culture plates containing X-gal and IPTG (both from Takara Bio). The insert sizes were determined for the colonies by PCRs using the primers LTR III and LC2 as well as Ex Taq (Takara Bio). Reaction mixtures were prepared according to the instructions attached to Ex Taq. PCRs were carried out as follows: denaturation at 95°C for 5 minutes; 30 cycles of 95°C for 60 seconds, 58°C for 45 seconds and 72°C for 90 seconds. After amplification, portions of the reaction mixtures were subjected to electrophoresis on 3.0%(w/v) agarose gel. Then, amplified fragments of various lengths were observed. Thus, it was shown that regions of integration sites derived from various clones were amplified according to the method of the present invention.

Additional 384 white colonies were selected and template DNAs for sequencing were prepared therefrom as follows. Each colony was inoculated into 40 µl of LB broth (1 g Trypton, 0.5 g yeast extract, 1 g sodium chloride per 100 ml) containing ampicillin and cultured at 37°C for 18 hours. 20 µl of 60% glycerol solution was added to the culture to prepare a glycerol stock (final concentration of 20%). Samples for sequencing were prepared from 0.5 µl each of the glycerol stocks using TempliPhi DNA Sequencing Template Amplification Kit (Amersham Biosciences) according to the instructions attached to the kit. The reaction mixtures were subjected to nucleotide sequence analyses using a sequencer MegaBACE 4000 (Amersham Biosciences). As a result, 115 human-derived sequences representing different integration sites were obtained. These results also show that amplified fragments derived from various clones were obtained according to the method of the present invention.

### Example 2

Analysis using a mixture of a polyclone-derived chromosomal DNA and a monoclone-derived chromosome as a sample (1)

### (1) Isolation of 293 cell clone having transferred retrovirus

100 µl of a 10²-fold dilution of the stock of amphoGFP virus supernatant prepared in Example 1(1) was added to 293 cells (5 x 10⁴ cells) in 1 ml of DMEM medium containing polybrene at a concentration of 8 µg/ml for infection. The medium was exchanged after 2 hours and the cells were cultured at 37°C for 24 hours in the presence of 5% CO₂. On the next day, the cells were replated into wells of a 96-well plate at a density of 1 cell per well in a medium containing G418 at a concentration of 500 µg/ml, and cultured at 37°C in the presence of 5% CO₂. Then, two cells (clones) that exhibited resistance to G418 were isolated and expanded to obtain 2 x 10⁶ cells for each clone. The two clones were designated as Clone 1 and Clone 2, and chromosomal DNAs were prepared from the respective clones according to the method as described in Example 1(3).

### (2) Amplification using monoclone-derived chromosomal DNA as template

Detection of sites at which the retrovirus vector had been integrated in the genomes of the clones was carried out according to the method as described in Example 1 (4) using, as a template, 0, 0.1, 1, 10, 50 or 100 ng of the chromosomal DNA from Clone 1 or Clone 2 prepared in Example 2(1). Portions of the final reaction mixtures were subjected to electrophoresis on 3.0%(w/v) agarose gel. The gel was stained with ethidium bromide for observing the amplification products. As a result, a fragment amplified from Clone 1 was observed using 1 ng of the chromosomal DNA, and a fragment amplified from Clone 2 was observed using 10 ng of the chromosomal DNA. Thus, it was shown that an amplified fragment derived from a specific clone can be detected with high sensitivity.

### (3) Analysis of sample containing clone-derived chromosomal DNA mixed with polyclone-derived chromosomal DNA

Samples each containing a total of 100 ng of chromosomal DNAs were prepared by mixing the chromosomal DNA prepared from 293R1 (used in Example 1) with the chromosomal DNA from Clone 1 or Clone 2 (prepared in Example 2(1)) at a concentration of 0, 0.1, 1, 10, 50 or 100%(w/w). Detection of the clone-derived DNA which had been mixed with the 293R1-derived chromosomal DNA was carried out according to the method as described in Example 1(4) using the sample as a template. Portions of the final reaction mixtures were subjected to electrophoresis on 3.0%(w/v) agarose gel for observing the amplification products. As a result, a fragment amplified from Clone 1 was observed even if the 293R1-derived chromosomal DNA was contained at a concentration of as low as 10%, and a fragment amplified from Clone 2 was observed even if the 293R1-derived chromosomal DNA was contained at a concentration of as low as 50%. Amplified fragments forming a broad band like those observed in Example 1 were observed using a sample containing the 293R1-derived chromosomal DNA.

In addition to the above, detection of a fragment amplified from the clone-derived DNA was carried out using, as a template, a mixture of a given amount (100 ng) of the 293R1-derived chromosomal DNA and 0, 0.1, 1, 10, 50 or 100 ng of the chromosomal DNA derived from Clone 1 or Clone 2 being added. The detection was carried out according to the method as described in Example 1(4). As a result, amplified fragments forming a broad band were observed for each reaction sample. Amplified fragments were detected using as little as 10 ng of the chromosomal DNA derived from Clone 1, and 50 ng of the chromosomal DNA derived from Clone 2, respectively. Thus, it was shown that an amplified fragment derived from a specific clone mixed with a polyclonal sample can be detected with high sensitivity.

### Example 3

Analysis using a mixture of a polyclone-derived chromosomal DNA and a monoclone-derived chromosome as a sample (2)

### (1) Amplification using a mixture of two clone-derived chromosomal DNAs as a template

Detection of fragments derived from integration sites in two genomes was examined according to the method as described in Example 1(4) using, as a template, a mixture of equal amounts (0, 1, 10, 50 or 100 ng each) of the chromosomal DNAs from Clone 1 and Clone 2 prepared in Example 2(1). Portions of the final reaction mixtures were subjected to electrophoresis on 3.0%(w/v) agarose gel for observing the amplification products. As a result, a fragment amplified from Clone 1 was observed using the mixture of 1 ng each of the chromosomal DNAs, and a fragment amplified from Clone 2 was observed using the mixture of 10 ng each of the chromosomal DNAs. Thus, it was shown that amplified fragments derived from the two clones can be detected with high sensitivity.

### (2) Amplification using a mixture of two clone-derived chromosomal DNAs mixed with a polyclone-derived one as a template

Samples each containing a total of 100 ng of chromosomal DNAs were prepared by mixing the chromosomal DNA prepared from 293R1 (used in Example 1) with a mixture of equal amounts of the chromosomal DNAs from Clone 1 and Clone 2 (prepared in Example 2(1)) at a concentration of 0, 0. 1, 1, 10, 50 or 100% (w/w). Detection of the two clone-derived DNAs which had been mixed with the 293R1-derived chromosomal DNA was carried out according to the method as described in Example 1(4) using the sample as a template.

Portions of the final reaction mixtures were subjected to electrophoresis on 3.0%(w/v) agarose gel for observing the amplification products. As a result, a fragment amplified from Clone 1 was observed using the sample containing the mixture of chromosomal DNAs at a concentration of 10%, and a fragment amplified from Clone 2 was observed using the sample containing the mixture of chromosomal DNAs at a concentration of 50%. Amplified fragments forming a broad band like those observed in Example 1 were observed using the sample containing the 293R1-derived chromosomal DNA.

In addition to the above, detection of fragments amplified from the clones was carried out using, as a template, a mixture of a given amount (100 ng) of the 293R1-derived chromosomal DNA and equal amounts (0, 0.1, 1, 10, 50 or 100 ng each) of the chromosomal DNAs derived from Clone 1 and Clone 2. The detection was carried out according to the method as described in Example 1(4). As a result, amplified fragments forming a broad band were observed in the agarose gel after electrophoresis for each reaction sample. A fragment amplified from Clone 1 was detected using the sample containing the mixture of 10 ng each of the chromosomal DNAs, and a fragment amplified from Clone 2 was detected using the sample containing the mixture of 50 ng each of the chromosomal DNAs. Thus, it was shown that amplified fragments derived from the two clones mixed with the polyclonal sample can be detected with high sensitivity.

### (3) Use of mixtures of two clone-derived chromosomal DNA at varying ratios as templates

Samples each containing a total of 100 ng of chromosomal DNAs were prepared by mixing the chromosomal DNAs from Clone 1 and Clone 2 (prepared in Example 2(1)) at a ratio by weight (Clone 1 : Clone 2, w:w) of 1:0, 4:1, 2:1, 1:1, 1:2, 1:4 or 0:1. Furthermore, samples containing 100 ng of the 293R1-derived chromosomal DNA (used in Example 1) in addition to the above-mentioned mixtures were prepared. Detection of fragments amplified from Clones 1 and 2 mixed together at varying ratios was carried out according to the method as described in Example 1(4) using the sample as a template.

Portions of the final reaction mixtures were subjected to electrophoresis on 3.0%(w/v) agarose gel for observing the amplification products. As a result, in case of the mixtures of Clone 1 and Clone 2, fragments amplified from the clones were observed with almost the same ratios as those of the original mixtures. In case of the samples containing the 293R1-derived chromosomal DNA being added, DNA fragments derived from Clones 1 and 2 were observed among amplified fragments forming a broad band. Variation in the amounts of the fragments amplified from Clones 1 and 2 was observed also in this case. Thus, it was shown that the two clones mixed with the polyclonal sample can be detected with high sensitivity according to the ratio.

### Example 4

Analysis using a mixture of chromosomal DNAs derived from four clones as a sample

### (1) Isolation of HL60 cell clone having transferred retrovirus

The plasmid pDON-GFP was transferred into a packaging cell GP+E-86, and a stock of amphoGFP virus supernatant was prepared from the resulting virus-producer cell according to the method as described in Example 1(1).

100 µl of a 10²-fold dilution of the stock of amphoGFP virus supernatant was added to HL60 cells (BioWhittaker, 5 x 10⁴ cells) in 1 ml of DMEM medium containing polybrene at a concentration of 8 µg/ml for infection. The medium was exchanged after 2 hours and the cells were cultured at 37°C for 24 hours in the presence of 5% CO₂. The cells were detached using trypsin, dispensed into six 10-cm tissue culture plates, and further cultured for 48 hours at 37°C in the presence of 5% CO₂. On the next day, the cells were replated into wells of a 96-well plate at a density of 1 cell per well in a medium containing G418 at a concentration of 500 µg/ml, and cultured at 37°C in the presence of 5% CO₂. The cells (clones) were replated into wells of a 24-well plate, cultured for four days, replated into wells of a 6-well plate, and cultured for four days. Then, four clones that exhibited resistance to G418 were isolated and expanded to obtain 1 x 10⁷ cells for each clone. The four clones were designated as Clone a, Clone b, Clone c and Clone d, and chromosomal DNAs were prepared from the respective clones according to the method as described in Example 1(3).

### (2) Reaction using a mixture of chromosomal DNAs derived from four clones as a template

Samples each containing 100 ng of one of the chromosomal DNAs prepared from Clones a-d in Example 4(1) or a mixture of equal amounts (0, 0.1, 1, 10, 25, 50 or 100 ng each) of the four chromosomal DNAs were prepared. Detection of bands resulting from amplification of the respective clones or mixture of bands derived from the four chromosomes was examined according to the method as described in Example 1(4) using the sample as a template.

Portions of the final reaction mixtures were subjected to electrophoresis on 3.0%(w/v) agarose gel for observing the amplification products. As a result, fragments amplified from Clones a, b, c and d were observed using the samples each containing only one of the chromosomal DNAs from them. In addition, four amplified fragments were detected using the mixture of four chromosomal DNAs. Regarding the sensitivity, detection could be carried out using the mixture containing about 1 ng each of them. Thus, it was shown that amplified fragments derived from the four clones (oligoclone) can be detected with high sensitivity.

### Example 5

### Increased detection sensitivity

Samples each containing a total of 100 ng of chromosomal DNAs were prepared by mixing the chromosomal DNA from 293R1 (prepared in Example 1(3)) with the chromosomal DNA from Clone 2 (prepared in Example 2(1)) at a concentration of 0, 0. 1, 1, 10, 50 or 100%(w/w). 0.5 µl of x10 NE buffer, 0.5 µl of a restriction enzyme Tsp509I (10 U/µl) and sterile water to a volume of 5 µl were added thereto. The mixture was incubated at 65°C for 1 hour. 25 µl of x2 PCR buffer, 5 µl of dNTP mix (2.5 mM each), 1 µl of a primer LTR I (0.1 pmol/µl) which has biotin added at its 5' end, 0.25 µl of Ex Taq and sterile water to a volume of 50 µl were added to the reaction mixture. The reaction mixture was placed in an automated gene amplification apparatus thermal cycler and subjected to a PCR as follows: denaturation at 95°C for 5 minutes; 50 cycles of 95°C for 60 seconds, 58°C for 45 seconds and 72°C for 90 seconds. Then, unreacted primers were removed as described in Example 1(4) to obtain 40 µl of a DNA solution.

Streptavidin-coated magnetic beads (40 µl, MPG streptavidin) were added to the solution. The mixture was allowed to stand for 1 hour at room temperature while occasionally mixing. A tube containing the reaction mixture was placed for 1 minute on a magnetic stand. Then, a supernatant was discarded to collect the beads, and the beads were washed. The washed beads were gently suspended in 14.1 µl of sterile water. 2 µl of x10 Klenow buffer, 2.4 µl of dNTP mix (2.5 mM each), 1 µl of a random primer (6mer, 100 pmol/µl) and 0.5 µl of Klenow fragment were added to the beads to prepare 20 µl of a mixture. The mixture was reacted at 37°C for 1 hour.

After reaction, the beads were washed, and 2 µl of linker cassette B (50 pmol/µl) (nucleotide sequences of two oligonucleotides constituting the linker cassette B are shown in SEQ ID NOS:9 and 10), 16 µl of Solution A (Takara Ligation kit ver. 1) and 2 µl of Solution B (Takara Ligation kit ver. 1) were added to the beads. The mixture was reacted at 16°C for 1 hour while occasionally mixing. After washing the beads once with sterile water, the beads were collected, suspended in 5 µl of 0.1 N sodium hydroxide, and allowed to stand at room temperature for 10 minutes. A supernatant (about 5 µl) was collected on the magnetic stand, and 1 µl of the supernatant was used for a PCR reaction as described in Example 1(4). The amplified fragment was confirmed by subjecting 5 µl of the reaction mixture of the second round of PCR (50 µl) to electrophoresis on 3.0%(w/v) agarose gel.

As a control, the procedure as described in Example 1(4) was carried out using the same template. As a result, a band amplified from Clone 2 was observed using a sample containing the chromosomal DNA from Clone 2 at a concentration of as little as 50% according to the method as described in Example 1(4). On the other hand, the band was observed using a sample containing the same at a concentration as little as 10% according to this method. Thus, the sensitivity of detection of an amplification product derived from a mixed clone could be increased.

### Example 6

### Comparison with conventional LAM PCR method (1)

### (1) Preparation of population of HL60 cells having transferred pDON-GFP

AmphoGFP virus-producer cells were constructed by transferring the plasmid pDON-GFP prepared in Example 4(1) into the packaging cell GP+E-86, and virus particles were collected from.

100 µl of a 10²-fold dilution of the stock of amphoGFP virus supernatant prepared in Example 4(1) was added to HL60 cells (5 x 10⁴ cells) in 1 ml of DMEM medium containing polybrene at a concentration of 8 µg/ml for infection. The medium was exchanged after 2 hours and the cells were cultured at 37°C for 24 hours in the presence of 5% CO₂. The cells were detached using trypsin, dispensed into six 10-cm tissue culture plates, and further cultured for 48 hours at 37°C in the presence of 5% CO₂.

The cells were then treated with trypsin, collected in a centrifuge tube, washed twice with PBS, and stored at -80°C. The group of transferred cells was designated as HL60R1. Analysis of HL60R1 using a FACS revealed a gene transfer efficiency of about 20%. Chromosomal DNA was prepared from HL60R1 (about 2.0 x 10⁶ cells) according to the method as described in Example 1(3).

### (2) Amplification of DNA of gene integration region

The conventional LAM PCR method was carried out as follows according to the method as described in Blood, 100:2737-2743 (2002). 10 µl of x10 PCR buffer (Qiagen), 8 µl of dNTP mix (2.5 mM each), 0.25 pmol of a primer LTR I which has biotin added at its 5' end, 0.5 µl of Taq polymerase (5 U/µl, Qiagen) and sterile water to a volume of 100 µl were added to 1, 10 or 100 ng of the genomic DNA prepared from HL60R1 in Example 6-(1). The reaction mixture was placed in an automated gene amplification apparatus thermal cycler (Takara Bio) and subjected to a PCR as follows: denaturation at 95°C for 5 minutes; 100 cycles of 95°C for 60 seconds, 60°C for 45 seconds and 72°C for 60 seconds; and incubation at 72°C for 10 minutes. An equal amount of Taq polymerase was added after the first 50 cycles, and the next 50 cycles were then carried out (a total of 100 cycles).

Magnetic beads (100 µl, corresponding to 200 µg) having immobilized streptavidin (MPG streptavidin) were added to the reaction mixture. The mixture was allowed to stand for 1 hour at room temperature while occasionally mixing. A tube containing the reaction mixture was placed for 1 minute on a magnetic stand. Then, a supernatant was discarded to collect the beads. The beads were gently mixed with 100 µl of BW buffer (5 mM tris-hydrochloride buffer (pH 7.5), 0.5 mM EDTA, 1.0 M sodium chloride). After standing on the magnetic stand, a supernatant was discarded in a similar manner. A similar procedure was carried out with 100 µl of sterile water to collect the beads (this procedure is called a beads washing procedure).

The washed beads were gently suspended in 14.1 µl of sterile water. 2 µl of x10 Klenow buffer, 2.4 µl of dNTP mix (2.5 mM each), 1 µl of a random primer (random 6mer, 100 pmol/µl, Takara Bio) and 0.5 µl of Klenow fragment (Takara Bio) were added to the beads to prepare 20 µl of a mixture. The mixture was reacted at 37°C for 1 hour while occasionally mixing to synthesize a complementary strand.

After reaction, the beads were washed. The collected beads were suspended in 17.5 µl of sterile water. 2 µl of x10 NE buffer and 0.5 µl of a restriction enzyme Tsp509I (10 U/µl) were added thereto. The mixture was incubated at 65°C for 1 hour while occasionally mixing. After the beads washing procedure, 2 µl of linker cassette A (50 pmol/µl), 16 µl of Solution A (Takara Ligation kit ver. 1) and 2 µl of Solution B (Takara Ligation kit ver. 1) were added to the beads. The mixture was reacted at 16 °C for 1 hour while occasionally mixing.

After reaction and washing the beads once with sterile water, the beads were collected, suspended in 5 µl of 0.1 N sodium hydroxide, and allowed to stand at room temperature for 10 minutes. A supernatant (about 5 µl) was collected on the magnetic stand, and 1 µl of the supernatant was used for a PCR reaction as follows. 10 µl of x10 Taq buffer, 8 µl of dNTP mix (2.5 mM each), 1 µl each of the primer LTR II (25 pmol/µl) and the primer LC1 (25 pmol/µl), 0.5 µl of Taq polymerase (Qiagen) and sterile water to a volume of 100 µl were added to 1 µl of the solution. The mixture was placed in an automated gene amplification apparatus thermal cycler and subjected to a PCR as follows: denaturation at 95°C for 5 minutes; 35 cycles of 95°C for 60 seconds, 60°C for 45 seconds and 72°C for 60 seconds; and incubation at 72°C for 10 minutes (the first round). Then, the second round of PCR was carried out using 0.2 µl of the reaction mixture of the first round of PCR as a template as well as the primer LTR III (25 pmol/µl) and the primer LC2 (25 pmol/µl). The conditions used for the second round of PCR were the same as those used for the first round of PCR.

Amplification of a DNA fragment flanking a retrovirus vector integration site was carried out also according to the method of the present invention as described in Example 1(4) using 1, 10 or 100 ng of the HL60R1 chromosomal DNA as a sample.

The amplification product was confirmed by subjecting 5 µl each of the reaction mixture to electrophoresis on 3.0%(w/v) agarose gel. As a result, amplified fragments of about 500 bp or less forming a broad band were observed in cases where 100 or 10 ng of the chromosomal DNA was used according to the method as described in Example 1(4). On the other hand, a band representing amplification from a specific clone was observed in a biased manner using 10 ng of the chromosomal DNA according to the conventional LAM PCR method as described in Blood. Based on these results, it was shown that DNA fragments derived from various gene-transferred cells can be amplified without being biased toward amplification from a specific clone according to the method of the present invention as described in Example 1(4) as compared with the conventional LAM PCR method even if the amount of template is little or the ratio of chromosomes having a transferred gene is little.

In addition, the same procedure was repeated. As a result, amplified fragments of about 500 bp or less forming a broad band were observed in cases where 100 or 10 ng of the chromosomal DNA was used according to the method as described in Example 1(4) as observed in the previous experiments. On the other hand, a band representing amplification from a specific clone was observed in a biased manner when not only 10 ng but also 100 ng of the chromosomal DNA was used according to the conventional LAM PCR method as described in Blood. These results show that analysis of retrovirus integration site can be carried out with reproducibility according to the method of the present invention as compared with the conventional LAM PCR method.

### Example 7

### Comparison with conventional LAM PCR method (2)

Amplification of DNA fragments of retrovirus integration sites were carried out according to the conventional LAM PCR method using the procedure as described in Example 6(2) and the following four types of templates: (A) 0, 0.1, 1, 10, 50 or 100 ng of the chromosomal DNA derived from Clone 1 prepared in Example 2(1); (B) 100 ng of the chromosomal DNA prepared from 293R1 (used in Example 1, polyclonal DNA) mixed with 0, 0.1, 1, 10, 50 or 100 ng of the DNA from Clone 1; (C) samples each containing a total of 100 ng of chromosomal DNAs prepared by mixing the chromosomal DNA prepared from 293R1 (used in Example 1) with 0, 0.1, 1, 10, 50 or 100 ng of the chromosomal DNA from Clone 1; and (D) samples each containing a total of 100 ng of chromosomal DNAs prepared by mixing the chromosomal DNA prepared from 293 cells without a transferred gene with 0, 0.1, 1, 10, 50 or 100 ng of the chromosomal DNA from Clone 1. Furthermore, DNA amplification was carried out according to the method of the present invention as described in Example 1(4) using the same templates for comparing the methods with each other.

Portions of the final reaction mixtures were subjected to electrophoresis on 3.0%(w/v) agarose gel for observing the amplification products. Amounts of the DNA derived from Clone 1 used for reaction mixtures with which amplified fragments were observed are shown in Table 1.

**Table 1**

| | Conventional | Present invention |
|---|---|---|
| (A) | ≥ 10 ng | ≥ 1 ng |
| (B) | ≥ 50 ng | ≥ 10 ng |
| (C) | ≥ 50 ng | ≥ 10 ng |
| (D) | ≥ 10 ng | ≥ 1 ng |

Based on the results, it was shown that the sensitivity of the method of the present invention upon detection of a clone predominantly present in various situations (in cases where a polyclonal DNA having a transferred gene or a chromosomal DNA without a transferred gene coexists in a reaction mixture) is 5- to 10-fold higher than that of the conventional method.

### Example 8

Comparison with the conventional LAM PCR method using a mixture of chromosomal DNAs derived from five clones as a sample

Five clones were selected from the gene-transferred cells prepared in Example 4(1). Amplified fragments of different lengths are generated from these clones upon amplification of DNA fragments containing retrovirus integration sites according to the method of the present invention. A mixture of chromosomal DNAs derived from the five clones was used as a template as follows.

The conventional LAM PCR method as described in Example 6(2) was compared with the method of the present invention as described in Example 1(4) using a mixture of equal amounts (1, 25 or 100 ng) of chromosomal DNAs derived from the five clones or a mixture further containing 100 ng of the chromosomal DNA from HL60R1 (prepared in Example 6-(1), polyclonal DNA).

Portions of the final reaction mixtures were subjected to electrophoresis on 3.0%(w/v) agarose gel for observing the amplification products. When only the mixture of chromosomal DNAs derived from the five clones was used as a template, fragments amplified from all of the five clones were observed according to the conventional method and the method of the present invention using 100 or 25ng of the template. When 1 ng of the template was used, fragments amplified from four of the clones were observed according to the method of the present invention, while DNA amplification was not observed at all according to the conventional LAM PCR method.

In case of coexistence of the polyclonal DNA in the template, fragments amplified from the five clones were observed according to the method of the present invention using 25 ng of the template. On the other hand, a fragment amplified from only one of the clones (not from the remaining four clones) was observed according to the conventional LAM PCR method using 25 ng of the template.

Based on the results, it was shown that, if plural clones predominantly present in a polyclone are to be detected, more sensitive detection (i.e., amplification reflecting the state of existing clones more properly) is possible according to the method of the present invention as compared with the conventional method.

### Example 9

### Comparison with the conventional LAM PCR method using chromosomal DNA derived from a peripheral blood clone from a bone marrow-reconstituted mouse as a sample

### (1) Preparation of retrovirus

An ecotropic retrovirus vector was prepared as follows. pGP and pE-eco contained in Retrovirus Packaging Kit Eco (Takara Bio) as well as the plasmid pDON-GFP prepared in Example 1(1) were transferred into 293 cells according to the instructions attached to the kit, the cells were cultured, and a culture supernatant was collected. The culture supernatant was filtered through a 0.45-micron filter (Millipore) to prepare a stock of ecoGFP virus supernatant, which was stored at -80°C until use. The infectivity titer of the ecoGFP virus supernatant against NIH/3T3 cells was 1.6 x 10⁶ infective viral particles/ml.

### (2) Preparation of mouse bone marrow cells

150 mg/kg of 5-fluorouracil (5-FU, Sigma) was intraperitoneally administered into a 7 weeks old C3H/He mouse (female, Japan SLC). Bone marrow was collected from femurs and tibias isolated after seven days. Mononuclear cells in a fraction prepared by centrifuging the thus obtained bone marrow cell fluid overlaid on Lymphocyte M (Daiichi Pure Chemicals) were used as mouse bone marrow cells.

The mouse bone marrow cells were added at a cell density of 1.45 x 10⁶ cells/ml to StemPro-34 SFM medium (Gibco) containing 50 ng/ml of thrombopoietin, 100 ng/ml of flt-3/flk-2 ligand, 100 ng/ml of stem cell factor, 5 µM 2-mercaptoethanol, 50 units/ml of penicillin and 50 µg/ml of streptomycin, and incubated at 37°C for 48 hours in the presence of 5% CO₂ for prestimulation. The prestimulated cells including those adhered to the vessel were collected by aspiration using a pipette.

### (3) Preparation of transformed bone marrow cells

The ecoGFP virus supernatant prepared in Example 9(1) was added to a plate (Takara Bio) coated with RetroNectin (Takara Bio), and the plate was incubated at 32°C. After four hours, the supernatant was removed by aspiration, and the mouse bone marrow cells (cell number, medium) were added thereto and incubated at 37°C for 22 hours in the presence of 5% CO₂. After incubation, the cells were collected by pipetting, washed once with saline, suspended in saline (Otsuka Pharmaceutical), and used as gene-transferred bone marrow cells.

### (4) Transplantation of transformed cells into mouse

The gene-transferred bone marrow cells (0.2 ml/mouse, corresponding to 5 x 10⁴ cells) were administered to 8 weeks old C3H/He mice (female) treated with lethal dose of X ray (900 Rad) through the tail veins. Peripheral bloods were collected from two mice 21 weeks after the cell transplantation. Chromosomal DNAs were prepared therefrom using QIAamp DNA Blood Mini Kit (Qiagen) according to the protocol attached to the kit and designated as Sample I and Sample II.

### (5) Analysis of gene integration site

The conventional LAM PCR method as described in Example 6(2) was compared with the method of the present invention as described in Example 1(4) using 100 ng of the Sample I or the Sample II as a template. The operations were conducted for both samples by two practitioners independently.

Portions of the final reaction mixtures were subjected to electrophoresis on 3.0%(w/v) agarose gel for observing the amplification products. As a result, a single major product as an amplified fragment was observed by one of the two practitioners according to the conventional LAM PCR method using the Sample I, while the major product was not observed by the other. No distinct amplified fragment was observed by both practitioners using the Sample II. On the other hand, amplified fragments as major products were distinctly observed using both samples in case of the method of the present invention. Minor products were also detected. Difference in amplified fragment pattern depending on the practitioners was not observed.

Based on the results, it was suggested that amplification with high sensitivity (i.e., in conformity with the situation of existing clones) is possible according to the present invention using an animal-derived sample. Furthermore, the reproducibility of the method of the present invention was not abolished by difference in practitioner.

### (6) Analysis of amplified fragment as minor product

It was confirmed that the amplified fragments as minor products amplified according to the method of the present invention in Example 9(5) were derived from cells having gene transferred using a retrovirus as follows. About 300-bp minor bands amplified according to the method of the present invention using the Sample I were recovered from gel and ligated to pT7BlueT vector according to the procedure as described in Example 1. Ten clones were selected from *Escherichia coli* cells transformed with the recombinant plasmids, and nucleotide sequences of fragments inserted in the plasmids harbored by the clones were analyzed. As a result, it was confirmed that all of the sequences were from mouse chromosomal DNA.

Based on the results, it was demonstrated that the minor products amplified according to the method of the present invention from reconstituted mouse blood cells were fragments amplified from gene-transferred cell clones. Since the minor products were not observed according to the conventional method, it was shown that the method of the present invention can be used to detect gene-transferred cell clones with sensitivity higher than the conventional method.

### Example 10

Amplification using reaction mixture containing dimethyl sulfoxide or tetramethylammonium chloride (TMAC)

The effect of addition of a compound to a reaction buffer was examined.

Samples each containing a total of 100 ng of a mixture of chromosomal DNAs as a template were prepared by mixing 34, 17 or 8.5 ng of the chromosomal DNA prepared from 293R1 (corresponding to 20, 10 or 5 ng of gene-transferred DNA based on the gene transfer efficiency of 59% for 293R1 prepared in Example 1) with the chromosomal DNA prepared from 293 cells without a transferred gene. DNA amplification according to the method as described in Example 1(4) was then carried out. In addition to the reaction mixture as described in Example 1(4), reaction mixtures that contain dimethyl sulfoxide (DMSO) at a final concentration of 6%, DMSO at a final concentration of 6% and TMAC at a final concentration of 10 mM, or TMAC at a final concentration of 10 mM in place of DMSO and betaine were prepared and used for reaction.

Portions of the final reaction mixtures were subjected to electrophoresis on 3.0%(w/v) agarose gel for observing the amplification products. As a result, amplified fragments of about 500 bp or less forming a broad band like those observed using the buffer as described in Example 1(4) were observed using the reaction mixture containing 6% DMSO. Using reaction mixtures containing 10 mM TMAC, increase in amplification efficiency as compared with the reaction mixture as described in Example 1(4) was observed regardless of the presence of DMSO. Amplified fragments forming a broad band were observed even if the amount of the gene-transferred DNA was small. The best result was observed using the reaction mixture that contained 10 mM TMAC but did not contain DMSO among the buffers.

Furthermore, amplification was conducted using a reaction mixture containing tetramethylammonium acetate in place of TMAC at the same concentration in the above-mentioned reaction mixture containing only 10 mM TMAC in PCR steps using the primers LTR II and LC1 or PCR steps using the primers LTR III and LC2. In this case, amplification almost equivalent to that observed using TMAC was observed.

### Industrial Applicability

According to the present invention, it is possible to accurately, sensitively and reproducibly monitor the extent of cells having an integrated gene in a population (polyclonal, oligoclonal or monoclonal), or the ratio of a specific cell in a population using a biological sample having various integration sites. Effects of conventional methods on analyses of integration sites in cells having a gene transferred using a retrovirus vector (monoclonal, oligoclonal, polyclonal) have been restricted due to their limits. The present invention can be applied to such analyses and, therefore, is very useful, for example, because highly precise monitoring in the field of gene therapy is expectable.

### Sequence Listing Free Text

SEQ ID NO:1; Gene encoding red-shifted green fluorescence protein.
SEQ ID NO:2; PCR primer LTR I to amplify a DNA fragment having LTR sequence.
SEQ ID NO:3; Oligonucleotide constituting linker-cassette A.
SEQ ID NO:4; Oligonucleotide constituting linker-cassette A.
SEQ ID NO:5; PCR primer LTR II to amplify a DNA fragment having LTR sequence.
SEQ ID NO:6; PCR primer LC1 to amplify a DNA fragment having linker-cassette A.
SEQ ID NO:7; PCR primer LTR III to amplify a DNA fragment having LTR sequence.
SEQ ID NO:8; PCR primer LC2 to amplify a DNA fragment having linker-cassette A.
SEQ ID NO:9; Oligonucleotide constituting linker-cassette B.
SEQ ID NO:10; Oligonucleotide constituting linker-cassette B.

## Claims

1. A method for analyzing a region of a chromosome-derived DNA flanking a retrovirus vector in a chromosomal DNA having the retrovirus vector being integrated, the method comprising:
(1) conducting a primer extension reaction using a primer that has a sequence complementary to a nucleotide sequence of an LTR of a retrovirus vector, and a chromosomal DNA having the retrovirus vector being integrated as a template to obtain a primer extension product;
(2) recovering the primer extension product obtained in step (1) and synthesizing a DNA that is complementary to the primer extension product to obtain a double-stranded DNA;
(3) adding a double-stranded oligonucleotide to the terminus of the double-stranded DNA obtained in step (2); and
(4) conducting a nucleic acid amplification reaction using the double-stranded DNA having the added oligonucleotide obtained in step (3) as a template, as well as a primer that has a sequence complementary to the nucleotide sequence of the LTR of the retrovirus vector and a primer that has a sequence complementary to the nucleotide sequence of the double-stranded oligonucleotide to obtain an amplification product,
wherein the primer extension reaction in step (1) and the nucleic acid amplification reaction in step (4) are conducted in the presence of a compound that lowers a Tm value of a double-stranded nucleic acid.

2. The method according to claim 1, wherein the primer extension reaction in step (1) is conducted using a polymerase chain reaction.

3. The method according to claim 1, wherein the nucleic acid amplification reaction in step (4) is conducted using a polymerase chain reaction.

4. The method according to claim 2 or 3, a DNA polymerase composition which is a mixture of a DNA polymerase that has a 3' exonuclease activity and a DNA polymerase that does not have a 3' exonuclease activity is used for the polymerase chain reaction.

5. The method according to claim 2 or 3, wherein the temperature cycle of the polymerase chain reaction used in step (1) or (4) consists of 95°C for 60 seconds; 58°C for 45 seconds; and 72°C for 90 seconds.

6. The method according to claim 1, wherein the compound that lowers a Tm value of a double-stranded nucleic acid is a substance selected from the group consisting of betaines, formamide, dimethyl sulfoxide and tetramethylammonium salts.

7. The method according to claim 6, wherein the compound that lowers a Tm value of a double-stranded nucleic acid is trimethylglycine.

8. The method according to claim 1, wherein a two-step polymerase chain reaction is used in step (4).

9. The method according to claim 1, wherein, in step (3), the double-stranded DNA obtained in step (2) is digested with a restriction enzyme and the double-stranded oligonucleotide is added to the generated terminus of the double-stranded DNA.

10. The method according to claim 1, wherein the chromosomal DNA in step (1) has been digested with a restriction enzyme beforehand.

11. The method according to claim 1, wherein a primer having a label is used in step (1) and the primer extension product is recovered utilizing the label in step (2).

12. The method according to claim 11, wherein a biotin-labeled primer is used.

13. The method according to claim 1, further comprising a step of determining a nucleotide sequence of the amplification product obtained in step (4).

14. The method according to claim 13, wherein the nucleotide sequence is determined using, as a template, a recombinant DNA in which the product obtained in step (4) is incorporated into a vector.

15. A kit for determining a nucleotide sequence of a chromosome-derived DNA flanking a retrovirus vector in a chromosomal DNA having the retrovirus vector being integrated, the kit containing:
(a) a primer that is capable of annealing to an LTR portion of a retrovirus;
(b) a linker cassette;
(c) a primer that is capable of annealing to the linker cassette of (b);
(d) a DNA polymerase;
(e) a restriction enzyme; and
(f) a buffer for a primer extension reaction which contains a compound that lowers a Tm value of a double-stranded nucleic acid.

16. The kit according to claim 15, wherein the DNA polymerase is a DNA polymerase composition which is a mixture of a DNA polymerase that has a 3' exonuclease activity and a DNA polymerase that does not have a 3' exonuclease activity.

17. The kit according to claim 15, wherein the compound that lowers a Tm value of a double-stranded nucleic acid is a substance selected from the group consisting of betaines, formamide, dimethyl sulfoxide and tetramethylammonium salts.

18. The kit according to claim 17, wherein the compound that lowers a Tm value of a double-stranded nucleic acid is trimethylglycine.

19. The kit according to claim 15, wherein the primer of (a) has a label for recovering a primer extension product.

20. A buffer for a primer extension reaction used for a method for analyzing a region of a chromosome-derived DNA flanking a retrovirus vector in a chromosomal DNA having the retrovirus vector being integrated, which contains a compound that lowers a Tm value of a double-stranded nucleic acid.

21. The buffer according to claim 20, wherein the compound that lowers a Tm value of a double-stranded nucleic acid is a substance selected from the group consisting of betaines, formamide, dimethyl sulfoxide and tetramethylammonium salts.

22. The buffer according to claim 21, wherein the compound that lowers a Tm value of a double-stranded nucleic acid is trimethylglycine.

23. Use of a compound that lowers a Tm value of a double-stranded nucleic acid for the manufacture of a buffer for a primer extension reaction used for a method for analyzing a region of a chromosome-derived DNA flanking a retrovirus vector in a chromosomal DNA having the retrovirus vector being integrated.

24. The use according to claim 23, wherein the compound that lowers a Tm value of a double-stranded nucleic acid is a substance selected from the group consisting of betaines, formamide, dimethyl sulfoxide and tetramethylammonium salts.

25. The use according to claim 24, wherein the compound that lowers a Tm value of a double-stranded nucleic acid is trimethylglycine.
